# EUROPÄISCHE PATENTANMELDUNG

(11) **EP 0 717 033 A2**
(43) Veröffentlichungstag der Anmeldung: **19.06.1996**
(21) Anmeldenummer: 95119454.7
(22) Anmeldetag: 11.12.1995
(51) Int. Cl.: C07C 315/00, C07C 317/44

(54) **Verfahren zur Herstellung von Methylsulfonylaromaten durch Methylierung von Arylsulfinsäuren unter Verwendung von Methylchlorid**

(30) Priorität: 15.12.1994 DE 4444694
(71) Anmelder: HOECHST AKTIENGESELLSCHAFT, D-65929 Frankfurt am Main (DE)
(72) Erfinder: Fülling, Gerd, Dr., D-65812 BadSoden (DE); Hörlein, Rolf, Dr., D-60529 Frankfurt (DE); Lerch, Ullrich, Dr., D-65719 Hofheim (DE); Worm, Manfred, Dr., D-65207 Wiesbaden (DE)

(57) **Zusammenfassung**

Beschrieben wird ein Verfahren zur Herstellung von Methylsulfonen der Formel IV 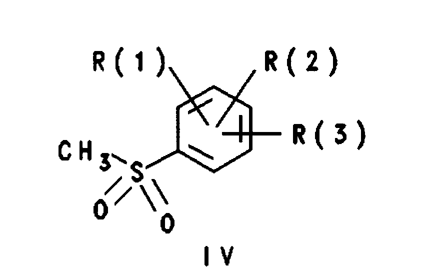
bei dem man auf eine aromatische Sulfinsäure der Formel III 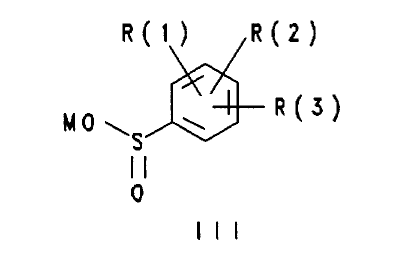
Methylchlorid einwirken läßt.

## Beschreibung

Die Erfindung betrifft ein Verfahren zur Herstellung von Methylsulfonen via S-Methylierung von Sulfinsäuren mit Methylchlorid, insbesondere von arylsubstituierten Sulfinsäuren zu Methylsulfonylaromaten.

Neben den wirtschaftlichen Vorteilen sind vor allem die geringere Toxizität von Methylchlorid im Vergleich zu den anderen Methylhalogeniden Methylbromid bzw. Methyliodid sowie das günstigere Umweltverhalten (Chlorid anstelle von Bromid bzw. Iodid im Abwasser) zu nennen.

Die Herstellung von Methylsulfonylaromaten aus den korrespondierenden arylsubstituierten Sulfinsäuren mit Methylhalogeniden ist an sich bekannt. Dazu wurden bislang Methylbromid und Methyliodid eingesetzt.

Sulfinat-Anionen sind ambidente Nucleophile (mesomere Formen I und II), die von einem Alkylierungsmittel sowohl am Schwefelatom unter Bildung von Methylsulfonen (Weg B) als auch am Sauerstoff unter Bildung von Sulfinsäuremethylestern (Weg A) alkyliert werden können. 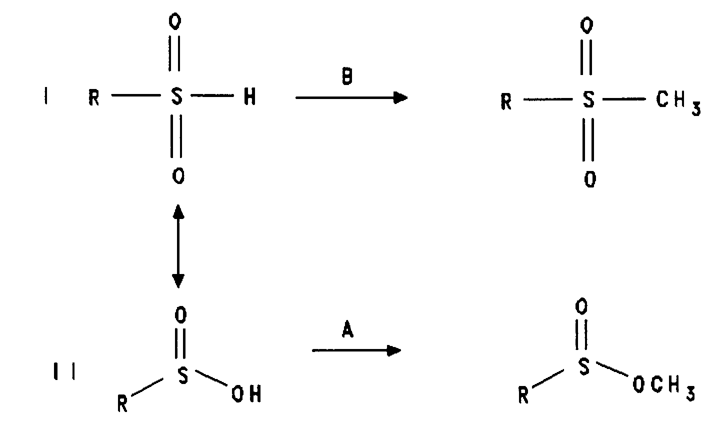
Nach der Theorie der harten und weichen Säuren und Basen [Chem. Rev. 75, 1-20, 1975] sollte das besser polarisierbare und damit im Vergleich zum Sauerstoff weichere Schwefelatom bevorzugt von weichen Methylierungsmitteln wie Methyliodid zu Methylsulfonen umgesetzt werden. Deshalb wird zur Überführung von Sulfinsäuren in Methylsulfone bevorzugt Methyliodid eingesetzt [z. B. DE 33 32 780 A 1, entsprechend US 4 661 636; DE 32 08 190 A 1, entsprechend CA 123 89 08; FR 25 04 924 B 1, bzw. entsprechender Derwent Report]. In einem Beispiel [DE 33 44 676 A 1, bzw. entsprechender Derwent Report] wird die Methylierung mit dem härteren Alkylierungsreagenz Methylbromid beschrieben. Während Iodide entsprechend einer Klassifizierung nach Becker [Einführung in die Elektronentheorie organisch-chemischer Reaktionen, VEB Deutscher Verlag der Wissenschaften, 3. Auflage, Berlin 1974, S. 191] als Verbindungen mit weichen Gruppen klassifiziert werden, zählen Chloride zu den Verbindungen mit harten Gruppen. Demnach sollte eine Methylierung unter Verwendung des harten Methylchlorids als Alkylierungsreagenz bevorzugt an der harten Stelle des ambidenten Sulfinat-Anions, also zur O-Alkylierung und demzufolge zur Bildung von Sulfinsäuremethylestern führen.

Zudem wird vom gleichen Autor [Becker, Einführung in die Elektronentheorie organisch-chemischer Reaktionen, VEB Deutscher Verlag der Wissenschaften, 3.

Auflage, Berlin 1974, S. 185] zitiert, daß auch die Abspaltungstendenz der nucleofugen Chloridgruppe deutlich schlechter ist als die der nucleofugen Gruppen Iodid und Bromid. So wird z. B. im direkten Vergleich zwischen Iodid und Bromid in Abhängigkeit vom Lösungsmittel ein Faktor 2 (protonische Lösungsmittel) bis 12 (aprotonische dipolare Lösungsmittel) beschrieben, mit dem Iodid schneller als Bromid ausgetauscht wird. Der Unterschied zwischen Bromid und Chlorid als Abgangsgruppe wird mit einem Faktor in den Reaktionsgeschwindigkeiten von sogar 50 (protonische Lösungsmittel) bis 250 (aprotonische dipolare Lösungsmittel) beschrieben, mit dem Bromid im Vergleich zu Chlorid schneller ausgetauscht wird.

Darauf ist vermutlich zurückzuführen, daß bislang im wesentlichen Methyliodid und nur in einem Fall Methylbromid, nicht aber Methylchlorid zur Methylierung von arylsubstituierten Sulfinsäuren herangezogen wurden. Nach den o. a. Ausführungen war zu erwarten, daß Methylchlorid aufgrund seiner Härte bevorzugt zur Alkylierung am Sauerstoff neigen sollte (Weg A) und aufgrund der deutlich geringeren relativen Abspaltungstendenz zu deutlich langsameren Reaktionsgeschwindigkeiten führen sollte.

Zudem wird in der Literatur beschrieben, daß die Methylierung von Arylsulfinsäuren zu schlechten Ausbeuten führt und große Überschüsse an Methylhalogenid eingesetzt werden müssen [Brown, J. Org. Chem. 56, 4974 - 4976 (1991)].

Überraschend wurde nun gefunden, daß bei Einwirkung von Methylchlorid auf aromatische Sulfinsäuren der Formel III durchaus die gewünschte S-Methylierung zu den entsprechenden Methylsulfonylaromaten beobachtet wurde und zudem mit einer Reaktionsgeschwindigkeit, die vergleichbar bis deutlich schneller als z. B. bei Einsatz von Methylbromid [DE 3344676 A1] als Alkylierungsmittel ist.

Außerdem braucht kein drastischer Überschuß am Methylchlorid eingesetzt zu werden, und es werden vergleichbar hohe Ausbeuten erzielt, obwohl dies aufgrund der Ausführungen in der Literatur nicht zu erwarten war.

Die Erfindung betrifft somit ein Verfahren zur Überführung von Sulfinsäuren (M = H) bzw. deren Salze (M = Li, Na, K bzw. 1/2 Mg, 1/2 Ca bzw. N-Kationen, wie Trialkylammonium) der Formel III in Methylsulfonylaromaten der Formel IV unter Verwendung des Alkylierungsmittels Methylchlorid. 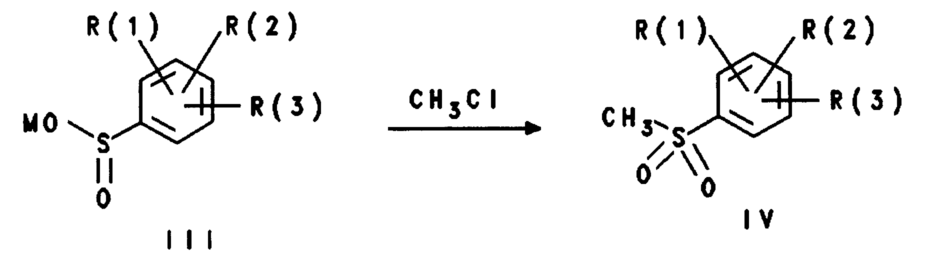
Als Aromat ist zwar in den Formeln nur ein Benzolring gezeichnet, es kommen aber ebenso Naphthalin, anellierte aromatische Systeme sowie Heteroaromaten in Betracht.

Der Aromat kann außer der Sulfinsäurefunktion mit M in der o. a. Bedeutung ein oder mehrfach substituiert sein mit folgenden Bedeutungen für R(1), R(2) oder R(3): Aryl ggf. weiter substituiert, geradkettiges oder verzweigtes Alkyl ggf substituiert, Halogen, Hydroxy und oder Carboxy (COOM mit M wie oben definiert), Thio- und Amino, ggf. geschützt etc.

Alkyl bedeutet, wenn nicht anders angegeben Alkyl mit 1, 2, 3, 4, 5, 6, 7 oder 8 C-Atomen, vorzugsweise Alkyl mit 1, 2, 3 oder 4 C-Atomen. Halogen steht, sofern nicht anders definiert, für F, Cl, Br oder I, bevorzugt für Cl und Br.

Das Verfahren wird mit Ausgangsverbindungen III ausgeübt, 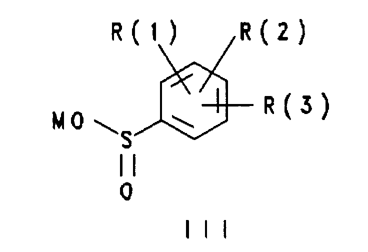
in denen bedeuten:
- R(1): H, (C₁-C₈)-Alkyl (geradkettig oder verzweigt), F, Cl, Br, I, OH, COOM, SH, NH₂, NR(4)R(5) oder OR(6);
R(4) und R(5) unabhängig voneinander
(C₁-C₅)-Alkyl, Phenyl oder Benzyl,
wobei die Phenylkerne unsubstituiert sind oder substituiert mit F, Cl, Br, I, (C₁-C₄)-Alkyl, (C₁-C₄)-Alkoxy, CF₃;
oder
R(4) und R(5) gemeinsam
4 oder 5 Methylengruppen, von denen eine CH₂-Gruppe durch Sauerstoff, S, NH, N-CH₃ oder N-Benzyl ersetzt sein kann;
R(6) Phenyl, Benzyl oder (C₁-C₈)-Alkyl (geradkettig oder verzweigt), wobei die Phenylkerne unsubstituiert sind oder substituiert mit F, Cl, Br, I, (C₁-C₄)-Alkyl, (C₁-C₄)-Alkoxy, CF₃;
- R(2): H, (C₁-C₈)-Alkyl, F, Cl, Br, I, OH, COOM, SH, NH₂, NR(4)R(5) oder OR(6); R(4) und R(5) unabhängig voneinander
(C₁-C₅)-Alkyl, Phenyl oder Benzyl,
wobei die Phenylkerne unsubstituiert sind oder substituiert mit F, Cl, Br, I, (C₁-C₄)-Alkyl, (C₁-C₄)-Alkoxy, CF₃;
oder
R(4) und R(5) gemeinsam
4 oder 5 Methylengruppen, von denen eine CH₂-Gruppe durch Sauerstoff, S, NH, N-CH₃ oder N-Benzyl ersetzt sein kann;
R(6) Phenyl, Benzyl oder (C₁-C₈)-Alkyl (geradkettig oder verzweigt),
wobei die Phenylkerne unsubstituiert sind oder substituiert mit F, Cl, Br, I, (C₁-C₄)-Alkyl, (C₁-C₄)-Alkoxy, CF₃;
- R(3): H, (C₁-C₈)-Alkyl, F, Cl, Br, I, OH, COOM, SH, NH₂, NR(4)R(5) oder OR(6); R(4) und R(5) unabhängig voneinander
(C₁-C₅)-Alkyl, Phenyl oder Benzyl,
wobei die Phenylkerne unsubstituiert sind oder substituiert mit F, Cl, Br, I, (C₁-C₄)-Alkyl, (C₁-C₄)-Alkoxy, CF₃;
oder
R(4) und R(5) gemeinsam
4 oder 5 Methylengruppen, von denen eine CH₂-Gruppe durch Sauerstoff, S, NH, N-CH₃ oder N-Benzyl ersetzt sein kann;
R(6) Phenyl, Benzyl oder (C₁-C₈)-Alkyl (geradkettig oder verzweigt), wobei die Phenylkerne unsubstituiert sind oder substituiert mit F, Cl, Br, I, (C₁-C₄)-Alkyl, (C₁-C₄)-Alkoxy, CF₃;
- M: Li, Na, K, Ca, Mg, Trialkylammonium.

Bevorzugt betrifft die Erfindung ein Verfahren, in der das Edukt die Struktur mit R(1) = Isopropyl oder Halogen, R(2) = COOM und R(3) = H und das Produkt die Struktur mit R(1) = Isopropyl oder Halogen, R(2) = COOM und R(3) = H aufweist.

Insbesondere betrifft die Erfindung ein Verfahren, in der das Edukt die Struktur der Formel V mit R(1) = 2-Isopropyl, R(2) = 5-COOM und R(3) = H und das Produkt die Struktur mit der Formel VI mit R(1) = 2-Isopropyl, R(2) = 5-COOM und R(3) = H aufweist. 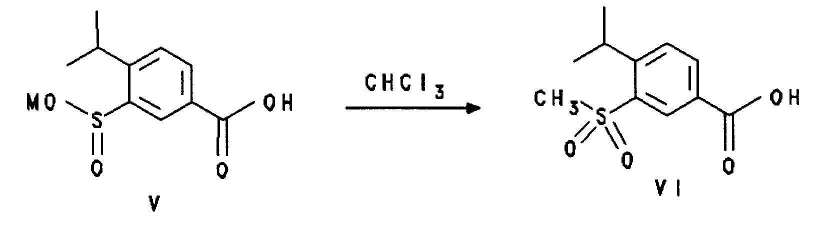
Ist unter den Resten R(1) bis R(3) eine weitere Funktion, wie R(2) = COOM, die methylierbar ist, so kann das Produkt als Methylester mit R(2) = COOCH₃ oder nach vorheriger Verseifung nach im Prinzip bekannten Verfahren durch saure oder alkalische Hydrolyse als freie Säure mit R(2) = COOH erhalten werden bzw. die Reaktion wird nach im Prinzip bekannten Verfahren so gesteuert, z. B. durch Wahl des geeigneten pH-Werts, daß die Zweitfunktion nicht methyliert wird.

Werden die freien Sulfinsäuren (M = H) eingesetzt, so überführt man die Sulfinsäurefunktion zunächst durch Zusatz von Base in das Sulfinat-Anion. Als Basen eignen sich anorganische Basen, wie LiOH, NaOH, KOH, Ca(OH)₂, Mg(OH)₂, oder organische Basen, wie Trialkylamine.

Als Lösungsmittel können protonische wie Wasser oder Alkohole oder aprotonische dipolare wie Amide, Sulfoxide, Ketone, Nitrile oder Ether bzw. Polyether oder ein Gemisch von beiden eingesetzt oder ein Gemisch aus beiden Lösungsmittelgruppen eingesetzt werden. In Einzelfall kann sogar das Alkylierungsmittel Methylchlorid selbst als Lösungsmittel dienen. So kommen z. B. die niederen Alkohole wie Methanol, Ethanol oder Isopropanol, sowie Aceton, 2-Butanon, Tetrahydrofuran, Ethylenenglykoldimethylether und höhere Homologe, Dimethylsulfoxid, Dimethylformamid, Dimethylacetamid, N-Methylpyrrolidon alleine oder als Gemisch mit Wasser in Betracht. In einer bevorzugten Form führt man die Methylierung mit Methylchlorid in Dimethylformamid, Dimethylacetamid oder N-Methylpyrrolidon (NMP) allein oder unter Zusatz von Wasser oder in Wasser allein um.

Die Reaktionstemperaturen lassen sich in einem weiten Bereich variieren und hängen unter anderem von den Löslichkeitseigenschaften der umzusetzenden Sulfinsäure ab. So sind grundsätzlich Reaktionstemperaturen von minus 20°C bis 150°C möglich, wobei im allgemeinen Reaktionstemperaturen von 20 bis 120°C zu bevorzugen sind. In einer besonders bevorzugten Durchführungsform wird man Reaktionstemperaturen von 60 bis 120°C wählen.

Falls in wäßrigen Lösungsmitteln gearbeitet wird, kann der eingestellte pH-Bereich zwischen pH 1 und pH 14 betragen. In einer bevorzugten Durchführungsform wird ein pH-Bereich von pH 4 - 13.5 eingestellt; bevorzugt für eine Reaktion in NMP ist ein pH-Wert von 4 - 8; für eine Reaktion in Wasser von 6 - 13.5, ganz besonders von 7 bis 12. Der pH-Wert wird zum Beispiel durch Zupumpen von NaOH oder durch Puffersysteme im gewünschten Bereich gehalten.

Das eingesetzte Methylchlorid kann in stöchiometrischen Mengen, bezogen auf alkylierbare funktionelle Gruppen, bis zu einem drastischen Überschuß eingesetzt werden. Ein bevorzugter Arbeitsbereich ist der Einsatz eines 2- bis 5-fachen Überschusses, bezogen auf die zu methylierende(n) Funktion(en).

Die Methylierung kann entweder im geschlossenen System unter Überdruck oder auch unter Normaldruck im offenen System durchgeführt werden, das heißt durch Einleiten des Methylchlorids in das zur Atmosphäre hin offene System. Es werden mit Methylchlorid vergleichbare Ausbeuten wie mit anderen Methylierungsmitteln erzielt. Dabei müssen keine unverhältnismäßig hohen Überschüsse an Methylchlorid eingesetzt werden.

Durch gezielte Steuerung des pH-Werts kann sowohl Me(SO₂)-Ar-COOH als auch Me(SO₂)Ar-COOCH₃ erhalten werden.

### Beispiel 1

### 4-Isopropyl-3-methylsulfonyl-benzoesäure - in NMP; drucklos

25 g (0,100 mol) 4-Isopropyl-3-sulfino-benzoesäure werden in 175 ml NMP suspendiert und mit 33-prozentiger Natronlauge auf pH 6 gestellt. Bei 90°C Innentemperatur werden innerhalb von 1,5 Stunden insgesamt 18,1 g (0,358 mol) Methylchlorid eingeleitet, wobei der pH-Wert durch kontinuierliches Zudosieren von 33-prozentiger NaOH bei pH 6 gehalten wird. Man rührt für 1,5 Stunden bei dieser Temperatur nach und stellt durch Zugabe von weiterer Natronlauge auf pH 12. Nach 1 Stunde wird schließlich mit konzentrierter Salzsäure auf pH < 1 gestellt, und der ausgefallenen Niederschlag wird abfiltriert und im Vakuum getrocknet.
Ausbeute: 19,6 g (81 %) 4-Isopropyl-3-methylsulfonyl-benzoesäure
Schmp.: 206°C
¹H-NMR (DMSO, 270 MHz): δ = 1,28 (6H, d); 3,28 (3H, s); 3,85 (1H, m); 7,82 (1H); 8,20 (1H); 8,45 (1H); 13,35 (1H, s, br)

### Beispiel 2

### 4-Isopropyl-3-methylsulfonyl-benzoesäure - in NMP; drucklos

27,3 g (0,109 mol) 4-Isopropyl-3-sulfino-benzoesäure-Mononatriumsalz werden in 150 ml NMP suspendiert und bei einer Innentemperatur 95°C innerhalb von 2 Stunden insgesamt 10,9 g (0,216 mol) Methylchlorid eingeleitet. Der pH-Wert wird dabei durch Zudosieren von 33-prozentiger Natronlauge zwischen pH 5,5 und 6,5 gehalten. Man läßt für 3 Stunden bei 95°C nachreagieren. Anschließend wird mit 33-prozentiger Natronlauge auf pH 12 gestellt und für 1 Stunde unter Rühren bei dieser Temperatur gehalten. Die Reaktionsmischung wird auf 450 g Eis/Wasser gegeben, für 1 Stunde gerührt und schließlich durch Zugabe von konzentrierter Salzsäure auf pH < 1 gestellt. Der Niederschlag wird abfiltriert und im Vakuum bei 50°C getrocknet.
Ausb.: 19,2 g (73 %) 4-Isopropyl-3-methylsulfonyl-benzoesäure
Schmp.: 206°C

### Beispiel 3

### 4-Isopropyl-3-methylsulfonyl-benzoesäuremethylester - in NMP; unter Druck

17,5 g (0,077 mol) 4-Isopropyl-3-sulfino-benzoesäure werden im Autoklaven in 200 ml NMP suspendiert und mit 8,5 g Natriumhydroxid versetzt. In den verschlossenen Autoklaven leitet man 200 g (3,960 mol) Methylchlorid ein und rührt für 24 Stunden bei 70°C. Die Reaktionslösung wird anschließend auf 500 ml Wasser gegossen, der ausgefallene Niederschlag abgesaugt und im Vakuum getrocknet.
Ausb.: 12,4 g (64 %) 4-Isopropyl-3-methylsulfonyl-benzoesäuremethylester
Schmp.: 150°C
¹H-NMR (DMSO, 270 MHz) δ = 1,26 (6H, d); 3,28 (3H, s); 3,85 (1H, m); 3,9 (3H, s); 7,85 (1H); 8,22 (1H); 8,46 (1H).

### Beispiel 4

### 4-Isopropyl-3-methylsulfonyl-benzoesäure; in Wasser, unter Druck

27.3 g (90%ig; 0.098 mol) 4-Isopropyl-3-sulfino-benzoesäure-Mononatriumsalz werden in einem 2 L-Laborautoklav in 150 mL E-Wasser suspendiert, dann wird mit 11.0 g Magnesiumoxid versetzt, es werden 27.0 g (0.545 mol) Methylchlorid eingeleitet und es wird auf 100° hochgeheizt. Der Druck steigt dabei von 4.5 auf 8.5 bar. Es wird für insgesamt 5 Stunden bei 95 - 105°C gerührt. Der Restdruck von 6 bar wird entspannt, und die Reaktionssuspension wird nach Zugabe vom 33 mL 32%iger Natronlauge für 1 Stunde bei 90°C gerührt. Die Reaktionsmischung wird schließlich mit 55 mL konzentrierter Salzsäure auf pH 1 gestellt, der Niederschlag abfiltriert und im vakuum bei 50°C getrocknet.
Ausbeute: 22.3 g (94%) 4-Isopropyl-3-methylsulfonyl-benzoesäure
Schmp. 209°C
HPLC: identisch mit Produkt aus Beispiel 1 und 2.

### Beispiel 5

### 4-Isopropyl-3-methylsulfonyl-benzoesäure; in Wasser, unter Druck

27.3 g (90%ig; 0.098 mol) 4-Isopropyl-3-sulfino-benzoesäure-Mononatriumsalz werden in einem 2-L-Laborautoklav in 218 mL E-Wasser suspendiert. Es werden zunächst 51 g 32%ige Natronlauge zudosiert (pH > 13), und anschließend werden 27.3 g (0.54 mol) Methylchlorid eingeleitet. Die Reaktionslösung wird auf 98°C Innentemperatur hochgeheizt. Der Druck steigt dabei auf 7 bis 8 bar. Es wird für insgesamt 7 Stunden bei 95 - 105°C gerührt, der Restdruck von 3 - 4 bar wird entspannt, und die Reaktionssuspension (pH 4.1) wird nach Zugabe von 32%iger Natronlauge (pH > 13) für 1 Stunde bei 100°C gerührt. Die Reaktionsmischung wird schließlich mit 32 g 37%iger Salzsäure auf pH 1 gesellt, es wird 2 Stunden bei ca 5 °C gerührt, der Niederschlag wird abfiltriert, mit Wasser gewaschen und im Vakuum bei 50°C getrocknet.
Ausbeute: 18.9 g(80%) 4-Isopropyl-3-methylsulfonyl-benzoesäure
Schmp. 209°C .
HPLC: identisch mit Produkt aus Beispielen 1 und 2.

## Patentansprüche

1. Verfahren zur Herstellung von Methylsulfonen der Formel IV 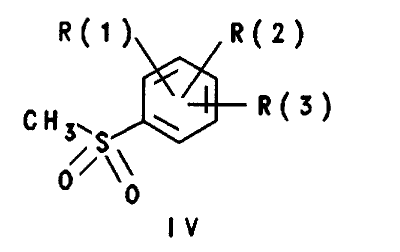 in welcher bedeuten:
R(1) H, (C₁-C₈)-Alkyl (geradkettig oder verzweigt), F, Cl, Br, I, OH, COOM, SH, NH₂, NR(4)R(5) oder OR(6);
R(4) und R(5) unabhängig voneinander
(C₁-C₅)-Alkyl, Phenyl oder Benzyl,
wobei die Phenylkerne unsubstituiert sind oder substituiert mit F, Cl, Br, I, (C₁-C₄)-Alkyl, (C₁-C₄)-Alkoxy, CF₃;
oder
R(4) und R(5) gemeinsam 4 oder 5 Methylengruppen, von denen eine CH₂-Gruppe durch Sauerstoff, S, NH, N-CH₃ oder N-Benzyl ersetzt sein kann;
R(6) Phenyl, Benzyl oder (C₁-C₈)-Alkyl (geradkettig oder verzweigt), wobei die Phenylkerne unsubstituiert sind oder substituiert mit F, Cl, Br, I, (C₁-C₄)-Alkyl, (C₁-C₄)-Alkoxy, CF₃;
R(2) H, (C₁-C₈)-Alkyl, F, Cl, Br, I, OH, COOM, SH, NH₂, NR(4)R(5) oder OR(6); R(4) und R(5) unabhängig voneinander
(C₁-C₅)-Alkyl, Phenyl oder Benzyl,
wobei die Phenylkerne unsubstituiert sind oder substituiert mit F, Cl, Br, I, (C₁-C₄)-Alkyl, (C₁-C₄)-Alkoxy, CF₃;
oder
R(4) und R(5)
gemeinsam 4 oder 5 Methylengruppen, von denen eine CH₂-Gruppe durch Sauerstoff, S, NH, N-CH₃ oder N-Benzyl ersetzt sein kann;
R(6) Phenyl, Benzyl oder (C₁-C₈)-Alkyl (geradkettig oder verzweigt),
wobei die Phenylkerne unsubstituiert sind oder substituiert mit F, Cl, Br, I, (C₁-C₄)-Alkyl, (C₁-C₄)-Alkoxy, CF₃;
R(3) H, (C₁-C₈)-Alkyl, F, Cl, Br, I, OH, COOM, SH, NH₂, NR(4)R(5) oder OR(6); R(4) und R(5) unabhängig voneinander
(C₁-C₅)-Alkyl, Phenyl oder Benzyl,
wobei die Phenylkerne unsubstituiert sind oder substituiert mit F, Cl, Br, I, (C₁-C₄)-Alkyl, (C₁-C₄)-Alkoxy, CF₃;
oder
R(4) und R(5) gemeinsam 4 oder 5 Methylengruppen, von denen eine CH₂-Gruppe durch Sauerstoff, S, NH, N-CH₃ oder N-Benzyl ersetzt sein kann;
R(6) Phenyl, Benzyl oder (C₁-C₈)-Alkyl (geradkettig oder verzweigt), wobei die Phenylkerne unsubstituiert sind oder substituiert mit F, Cl, Br, I, (C₁-C₄)-Alkyl, (C₁-C₄)-Alkoxy, CF₃;
M Li, Na, K, Ca, Mg, Triethylammonium,
via S-Methylierung von Sulfinsäuren mit einem Methylhalogenid,
dadurch gekennzeichnet,
daß man auf eine aromatische Sulfinsäure der Formel III 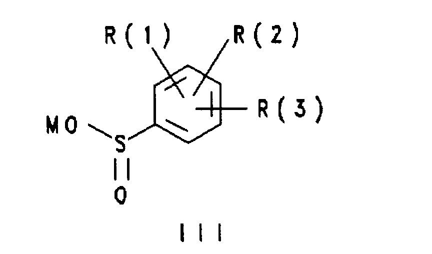 in welcher R(1), R(2), R(3) und M die oben angegebenen Bedeutungen haben,
Methylchlorid einwirken läßt.

2. Verfahren nach Anspruch 1, dadurch gekennzeichnet, daß darin bedeuten:
R(1) Isopropyl, F, Cl, Br, I;
R(2) COOM;
R(3) Wasserstoff;
M Wasserstoff, Li, Na, K, Ca, Mg, Triethylammonium.

3. Verfahren nach Anspruch 1, dadurch gekennzeichnet, daß darin bedeuten:
R(1) 2-Isopropyl;
R(2) 5-COOM;
R(3) Wasserstoff;
M Wasserstoff, Li, Na, K, Ca, Mg, Triethylammonium.
